# FASCICULE DE BREVET EUROPEEN

(11) **EP 0 869 107 B1**
(45) Date de publication et mention de la délivrance du brevet: **25.07.2001**
(21) Numéro de dépôt: 98400735.1
(22) Date de dépôt: 30.03.1998
(51) Int. Cl.: C07C 11/02, C07C 41/06

(54) **Procédé de production d'alpha oléfine, d'oléfine tertiaire et/ou d'éther à partir de coupe hydrocarbonée insaturée**
Verfahren zur Herstellung von alpha-Olefinen, tertiären Olefinen und/oder Ethern aus einer ungesättigten Kohlenwasserstofffraktion
Process for the preparation of alpha olefins, tertiary olefins and/or ethers from an insaturated hydrocarbon fraction

(30) Priorité: 02.04.1997 FR 9704117
(43) Date de publication de la demande: 07.10.1998
(73) Titulaire: INSTITUT FRANCAIS DU PETROLE, 92852 Rueil-Malmaison Cedex (FR)
(72) Inventeur: Marion, Marie-Claire, 69100 Villeurbanne (FR); Coupard, Vincent, 69002 Lyon (FR); Forestiere, Alain, 69390 Vernaison (FR); Travers, Philippe, 92500 Rueil Malmaison (FR); Viltard, Jean-Charles, 26000 Valence (FR)
(74) Mandataire: Andréeff, François

(56) Documents cités:
- FR-A- 2 527 201
- US-A- 4 447 668
- US-A- 5 567 860

## Description

L'invention concerne un procédé intégré de synthèse d'alpha oléfine pure, d'oléfine tertiaire pure et éventuellement d'éther alkylique tertiaire à partir d'au moins une coupe hydrocarbonée insaturée contenant au moins une alpha oléfine et au moins une oléfine tertiaire éthérifiable; comprenant une étape d'éthérification de ladite coupe hydrocarbonée par au moins un alcool dans laquelle on forme un produit comprenant au moins un éther alkylique tertiaire que l'on envoie dans une zone de séparation à partir de laquelle on récupère une fraction hydrocarbonée appauvrie en éther alkylique tertiaire contenant ladite alpha oléfine et une fraction enrichie en éther alkylique tertiaire dont au moins une partie est éventuellement récupérée et l'autre partie ou la totalité est envoyée dans une zone de décomposition d'éther(s) alkylique(s) tertiaire(s) à partir de laquelle on récupère un produit comprenant au moins une oléfine tertiaire de haute pureté.

L'invention concerne en particulier un procédé de production de butène-1 de haute pureté, d'isobutène de haute pureté et de préférence d'éther méthylique de l'alcool tertiobutylique (MTBE initiales anglaises de Methyl-Tertio-Butyl-Ether), à partir d'une coupe C4 hydrocarbonée contenant du butène-1 et de l'isobutène, comprenant la formation à partir de cette coupe et de méthanol d'un produit contenant du MTBE, puis la séparation à partir de ce produit d'une fraction hydrocarbonée appauvrie en MTBE contenant ledit butène-1 et en une fraction enrichie en MTBE dont au moins une partie est éventuellement récupérée et l'autre partie ou la totalité est envoyée dans une zone de décomposition du MTBE à partir de laquelle on récupère un produit comprenant du méthanol et de l'isobutène que l'on envoie dans une zone de fractionnement à partir de laquelle on obtient un produit enrichi en alcool méthylique, que l'on peut éventuellement recycler à la synthèse du MTBE et un produit enrichi en isobutène purifié. La suite de la description de la présente invention et tout particulièrement les conditions opératoires sont données à titre indicatif pour la synthèse d'isobutène et la récupération du butène-1 à partir de coupes hydrocarbonées contenant essentiellement des hydrocarbures à 4 atomes de carbone (notamment du butène-1 et de l'isobutène) par synthèse et décomposition du MTBE. Le procédé de la présente invention s'applique également à la synthèse d'autre(s) oléfine(s) tertiaire(s) et à la récupération d'autre(s) alpha-oléfine(s) à partir d'autre(s) coupe(s) hydrocarbonée(s) contenant ces oléfines. Le procédé de la présente invention comprend une étape de synthèse d'au moins un éther alkylique tertiaire (par exemple ETBE (Ethyl-Tertio-Butyl-Ether), ETAE (Ethyl Tertio-Amyl-Ether), TAME (Tertio-Amyl-Methyl-Ether), isopropyl-tertio-butyléther) et sa décomposition .

Dans le contexte actuel des essences reformulées, la production des éthers tels que le MTBE, l'ETBE ou le TAME, pour augmenter l'indice d'octane, ne cesse de croître. Par ailleurs, dans le domaine de la chimie et pétrochimie et en particulier dans le domaine des polymères (poly-isobutène, polyéthylènes spéciaux...) les besoins en oléfines en particulier il existe des demandes en isobutène et en butène-1 de haute pureté sont également croissants. Hors, l'isobutène et le butène-1 qui ont un point d'ébullition très proche (respectivement -6,9 et -6,3 °C), ne peuvent pas être séparés facilement par simple distillation.

Un des moyens pour séparer aisément le butène-1 de l'isobutène est de transformer sélectivement l'isobutène en éther, par exemple en MTBE ou ETBE. L'étape d'éthérification permet alors d'isoler l'isobutène, sous forme d'éther alkylique tertiaire. L'isobutène peut ensuite, par la réaction inverse de décomposition de l'éther, être produit à une (très) haute pureté. Selon ce schéma, et à condition d'effectuer une éthérification poussée de l'isobutène, le butène-1 peut être produit avec une pureté très élevée.

La production d'isobutène de haute pureté par craquage du MTBE est aussi bien adaptée pour des petites capacités que pour des grosses capacités. De plus, cette voie bénéficie de toute l'infrastructure relative à l'importance croissante des éthers dans les essences reformulées. De nombreuses raffineries, partout dans le monde, possèdent des installations de productions de MTBE par exemple. D'autre part il y a également un marché d'échange du MTBE au niveau mondial. Cela signifie que la production d'isobutène de haute pureté, à partir de MTBE, peut être mise en oeuvre aisément partout dans le monde, y compris en dehors des raffineries.

L'idée de produire de l'isobutène par décomposition d'éther, et plus particulièrement du MTBE, est connue, mais les procédés de mise en oeuvre proposés par l'art antérieur présentent certains inconvénients.

Ainsi dans le procédé développé par SUMITOMO, décrit par exemple dans la demande de brevet EP-A-68 785, la réaction de décomposition du MTBE est réalisée en phase liquide, en présence d'un catalyseur solide acide de type résine échangeuse d'ions. Deux flux de produit sont obtenus : l'isobutène et le méthanol. Tel que le schéma est décrit, l'isobutène est directement obtenu en tête d'une colonne à distiller sans autre étape de purification. L'isobutène ainsi obtenu contient un certain nombre d'impuretés à commencer par une petite fraction de méthanol qui est distillée par azéotropie, et du diméthyléther (DME) composé volatil formé par condensation du méthanol en présence d'un catalyseur acide. Il est probable que la pureté de l'isobutène est alors insuffisante pour une utilisation telle que la fabrication de polyisobutène ou d'autres copolymères. En outre, aucun moyen ne permet apparemment d'éviter l'accumulation d'impuretés lourdes telles que les dimères de l'isobutène ou l'éther méthylique de l'alcool butylique secondaire (MSBE), ce qui à terme se traduit fatalement par une baisse de pureté des produits.

Dans le procédé développé par ERDOLCHEMIE, décrit par exemple dans le brevet US-A-4 409 421, la purification de l'isobutène, qui consiste à éliminer l'alcool résiduel entraîné avec l'oléfine tertiaire, est réalisée par adsorption. Cette méthode présente l'inconvénient d'avoir à régénérer régulièrement l'adsorbant. En outre la récupération de la majeure partie de l'alcool issu de la décomposition n'est pas solutionnée.

Plus récemment, la même société décrit, dans le brevet US-A-5 095 164, la mise en oeuvre de la réaction de décomposition dans un appareillage de distillation. Le catalyseur est alors placé dans le fond de la colonne au niveau du rebouilleur. Cette mise en oeuvre particulière est limitative sur le plan de la température réactionnelle, directement imposée par la nature de l'éther et la pression opératoire. En outre, elle favorise vraisemblablement la formation de sous-produits de réaction tels que la formation de dimères de l'isobutène et/ou la formation de diméthyléther. A ce propos, la qualité et/ou le devenir des produits ne sont pas clairement explicités.

Par ailleurs, la société BASF décrit, dans le brevet US-A-4 287 379, un schéma intégrant à la fois l'étape de synthèse de l'éther, sa séparation, puis l'étape de décomposition de l'éther pour produire l'isobutène. Toutefois, pour éviter certaines étapes de purification, l'éthérification est faite avec un alcool en C3 ou C4, ce qui est un inconvénient majeur face au marché international du MTBE.

Le procédé de production d'isobutène décrit dans le brevet US 4,447,668 prévoit, en aval de l'étape de décomposition de l'éther, l'introduction, dans une zone de lavage à l'eau, de la totalité de l'effluent issu de l'étape de décomposition. Dans un tel schéma, il est inévitable qu'une partie de l'éther soit entraînée avec la fraction aqueuse récupérée en fond de la colonne de lavage, d'où une perte substantielle de l'éther non décomposé, qui ne peut pas être recyclé. D'autre part, l'effluent de la zone de décomposition étant introduit dans sa totalité dans la colonne d'extraction à l'eau, celle-ci doit présenter des caractéristiques de dimensionnement importantes.

Nous pouvons également signaler les deux schémas de principe proposés par la société SNAMPROGETTI dans Chemical Economy & Engineering Review, vol. 14 n° 6 Juin 1982, incluant à la fois l'étape de synthèse du MTBE et l'étape de décomposition du MTBE pour la production d'isobutène. Toutefois ces schémas ne prévoient pas la récupération d'alpha oléfines et en particulier la production de butène-1 de haute pureté.

Il a de plus été décrit dans le brevet US-A-4324924 un procédé comprenant la synthèse de MTBE à partir d'une coupe C₄ contenant de l'isobutène et du butène-1 comprenant la séparation du produit issu du réacteur d'éthérification en une fraction comprenant du MTBE et en une fraction contenant les hydrocarbures non convertis et du méthanol qui est ensuite envoyée dans un deuxième réacteur d'éthérification avant d'être à son tour scindée en une fraction contenant du méthanol et en une fraction essentiellement hydrocarboné que l'on lave pour éliminer les traces de méthanol qu'elle contient et que l'on récupère. Ce procédé ne comprend pas la production simultanée d'isobutène de haute pureté et de butène-1 de haute pureté. Par ailleurs sa mise en oeuvre est délicate car elle implique la présence d'un réacteur intermédiaire d'éthérification entre deux zones de distillation sans réel contrôle de la stoechiométrie alcool/oléfine tertiaire à l'entrée de ce deuxième réacteur.

Il a encore été décrit dans le brevet US-A-4797133 un procédé de récupération de butène-1 de haute pureté à partir d'une coupe C₄ en contenant et contenant aussi de l'isobutène. Ce procédé comprend l'éthérification de l'isobutène, la séparation du produit issu de la zone d'éthérification en une fraction enrichie en MTBE et en une fraction appauvrie en MTBE contenant du butène-1. Cette dernière fraction est envoyée dans une zone de lavage à l'eau à partir de laquelle on obtient une fraction appauvrie en méthanol contenant le butène-1 que l'on envoie dans une colonne à distiller à partir de laquelle on obtient du butène-1 de haute pureté. La fraction contenant du MTBE est quant à elle envoyée en partie dans une zone d'alkylation ou envoyée en partie dans les fractions carburant. Ce procédé ne comprend pas la production simultanée d'isobutène de haute pureté et de butène-1 de haute pureté.

Le procédé selon l'invention permet de remédier aux inconvénients des systèmes précités . Il concerne un procédé intégré de production d'alphaoléfine(s) de haute pureté, d'oléfine(s) tertiaire(s) caractérisée(s) par une très haute pureté et le plus souvent également d'éther(s) alkylique(s) tertiaire(s), à partir d'une coupe hydrocarbonée contenant au moins une alpha-oléfine et au moins une oléfine tertiaire éthérifiable, par une méthode comprenant la formation puis la décomposition d'un éther alkylique tertiaire. Ce procédé est donc également un procédé adapté à la valorisation des oléfines tertiaires et des alpha-oléfines contenues dans les coupes hydrocarbonées sous forme d'alpha-oléfines purifiées, d'oléfines tertiaires purifiées et/ou d'éthers alkyliques tertiaires. Les oléfines sont utilisables en particulier dans la formation de polymères et/ou de copolymères. Les éthers alkyliques tertiaires sont utilisables en particulier comme additifs dans les carburants pour moteurs à combustion.

L'invention concerne un procédé comprenant la synthèse d'éther(s) alkylique tertiaire(s), en particulier de MTBE ou ETBE, et la décomposition d'au moins une partie de ces éther(s) alkylique(s) tertiaire(s), en particulier de MTBE ou ETBE, pour la production d'oléfine(s) tertiaire(s), d'isobutène en particulier, de haute pureté. Dans le cas de la décomposition d'autres éthers on peut obtenir un mélange contenant une pluralité d'oléfines tertiaires. Ainsi dans le cas de la décomposition du TAME, on obtient un mélange contenant du méthyl-2 butène-1 et du méthyl-2 butène-2.

Outre les zones de réaction proprement dites, le procédé selon l'invention comprend des zones de purification ou récupération ou recyclage des divers produits de façon à optimiser la valorisation des produits mis en oeuvre et minimiser les pertes.

Le procédé de la présente invention peut comprendre diverses variantes qui permettent en particulier d'améliorer la qualité de l'oléfine tertiaire récupérée et qui sont décrites ci-après. Ces variantes peuvent être réalisées séparément ou simultanément, soit en totalité, soit associées par deux ou plus.

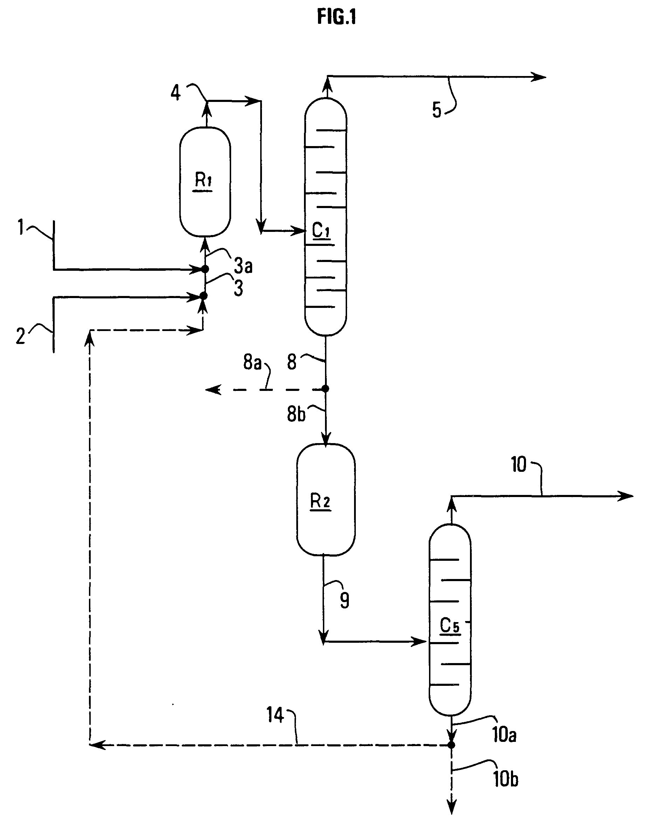

Les figures 1 à 5 sont des schémas de principe illustrants chacune une des multiples variantes de mise en oeuvre de la présente invention. Les traits en pointillés montrent certaines des diverses options possibles tant en ce qui concerne les options de recyclage, qu'en ce qui concerne les appareillages optionnels dans la variante considérée. La description de la présente invention est faite en liaison avec ces figures dans le but d'en faciliter la compréhension. Sur ces figures les organes similaires sont désignés par les même chiffres et lettres de référence. La figure 1 qui illustre la forme la plus générale de la présente invention est décrite ci-après.

La présente invention concerne un procédé de production d'alpha oléfine pure, d'oléfine tertiaire pure et éventuellement d'éther alkylique tertiaire pur à partir de coupe hydrocarbonée insaturée contenant au moins une alpha oléfine et au moins une oléfine tertiaire éthérifiable, ledit procédé étant caractérisé en ce qu'il comprend :
a) une étape de formation d'au moins un éther alkylique tertiaire par mise en contact dans une zone (R1) réactionnelle, comprenant généralement au moins un réacteur et contenant un catalyseur d'éthérification, d'une coupe hydrocarbonée insaturée contenant au moins une alpha oléfine et au moins une oléfine tertiaire éthérifiable avec au moins un alcool, ayant généralement de 1 à 6, et de préférence de 1 à 4 atomes de carbone par molécule, et étant généralement un alcool primaire ou secondaire, de préférence un alcool primaire, et de préférence l'alcool méthylique ou l'alcool éthylique ou l'isopropanol, de façon encore plus préféré l'acool methylique ou l'acool éthylique, généralement dans des conditions de transformation d'au moins une partie de l'oléfine tertiaire en éther alkylique tertiaire,
b) une étape de fractionnement d'au moins une partie du produit issu de l'étape a) dans une zone de fractionnement (C1) permettant d'obtenir d'une part une fraction organique (O1) appauvrie en éther alkylique tertiaire, et de préférence ne contenant pratiquement pas d'éther alkylique tertiaire, et contenant l'alpha oléfine (par exemple le butène-1), et en une fraction organique (E1) enrichie en éther alkylique tertiaire formé au cours de l'étape a) et de préférence contenant pratiquement la totalité dudit éther alkylique tertiaire,
c) une étape de décomposition, d'au moins une partie de l'éther alkylique tertiaire contenu dans la fraction organique (E1) de l'étape b), dans une zone réactionnelle (R2) comprenant généralement au moins un réacteur et contenant un catalyseur de décomposition dudit éther, ladite étape étant effectuée dans des conditions permettant la décomposition au moins partielle dudit éther alkylique tertiaire en un produit (P1) contenant au moins un alcool et au moins une oléfine tertiaire, et
d) une étape de fractionnement d'au moins une partie du produit (P1) issu de l'étape c), dans une zone de fractionnement (C5) permettant d'obtenir d'une part une fraction (B1) contenant la majeure partie de l'oléfine tertiaire et une fraction mineure d'alcool et éventuellement des composés légers éventuellement contenus initialement dans ladite partie du produit de (P1), et d'autre part une fraction (A1) contenant la majeure partie de l'alcool formé dans l'étape c), éventuellement de l'éther non décomposé dans l'étape c) et éventuellement des composés lourds contenus initialement dans ladite partie du produit (P1).

Les coupes hydrocarbonées que l'on utilise dans le cadre de la présente invention contiennent au moins une oléfine tertiaire, au moins une alpha oléfine et en général d'autres composés hydrocarbonés saturés ou insaturés tels que par exemple d'autres oléfines (par exemple des oléfines linéaires), des paraffines (linéaires ou branchés), éventuellement une faible proportion d'eau, et/ou des oxydes de carbone. Les oléfines que l'on envisage de préparer selon la présente invention sont pour les oléfines tertiaires purifiées des composés dont un atome de carbone de la liaison oléfinique est branché et pour les alpha oléfines purifiées des composés linéaires dont l'atome de carbone de la liaison oléfinique est l'atome terminal. Ces composés ont habituellement de 4 à 10 atomes de carbone par molécule, de préférence de 4 à 8 atomes de carbone par molécule et le plus souvent de 4 à 6 atomes de carbone par molécule. On peut ainsi citer pour ce qui est des oléfines tertiaires l'isobutène, le méthyl-2 butène-1, le méthyl-2 butène-2, les héxènes tertiaires, les octènes tertiaires et les décènes tertiaires. On peut citer les coupes C₄ et/ou C₅ issues du raffinage ou de la pétrochimie, les coupes C₄ et/ou C₅ de vapocraquage, habituellement après extraction des diènes, les coupes C₄ et/ou C₅ de craquage catalytique, les coupes issues d'isomérisation (hydroisomérisation ou isomérisation squelettales), les coupes obtenues par déshydrogénation de paraffines.

Dans une forme particulière de mise en oeuvre du procédé selon l'invention, une partie de la fraction organique (E1) contenant de l'éther alkylique tertiaire peut être envoyée aux fractions carburant moteur et l'autre partie envoyée à l'étape c) de décomposition de l'éther. Cette forme de mise en oeuvre de la présente invention est en particulier illustrée sur les figures 1 à 4 (ligne 8a). En ce qui concerne la figure 1 la charge hydrocarbonée contenant au moins une alpha oléfine et au moins une oléfine tertiaire est introduite par les lignes 1 et 3a dans la zone d'éthérification R1. Dans cette zone R1 on introduit également par les lignes 2, 3 et 3a un appoint d'alcool et éventuellement par les lignes 14, 3, et 3a de l'alcool recyclé. Cette zone contient un catalyseur acide d'éthérification. Le produit obtenu par éthérification de la coupe hydrocarboné est envoyé par la ligne 4 dans la zone de fractionnement (C1) permettant d'obtenir d'une part une fraction organique (O1) appauvrie en éther alkylique tertiaire contenant le butène-1 recherché que l'on récupère par la ligne 5 et en une fraction organique (E1) contenant l'éther formé qui sort par la ligne 8. Une partie de ladite fraction (E1) est envoyée par la ligne 8a aux fractions carburant moteur et l'autre partie est envoyé par la ligne 8b dans la zone R2 de décomposition de l'éther. Par la ligne 9 on récupère un produit (P1) contenant au moins une oléfine tertiaire, au moins un alcool, éventuellement de l'éther non décomposé dans l'étape c), éventuellement des composés légers et éventuellement des composés lourds, et on envoie ledit produit (P1) en partie, de préférence en majeure partie, par la ligne 9 dans la zone de fractionnement (C5) à partir de laquelle on obtient par la ligne 10 une fraction (B1) contenant la majeure partie de l'oléfine tertiaire, éventuellement une fraction mineure d'alcool et éventuellement des composés légers éventuellement contenus initialement dans ladite partie de produit et on obtient par la ligne 10a une fraction (A1) contenant la majeure partie de l'alcool formé dans l'étape c), éventuellement de l'éther non décomposé dans l'étape c) et éventuellement des composés lourds contenus initialement dans ladite partie de produit (P1). Au moins une partie de ladite fraction (A1) peut être recyclée par les lignes 14, 3 et 3a dans le réacteur (R1) ou être évacuée par les lignes 10a et 10b.

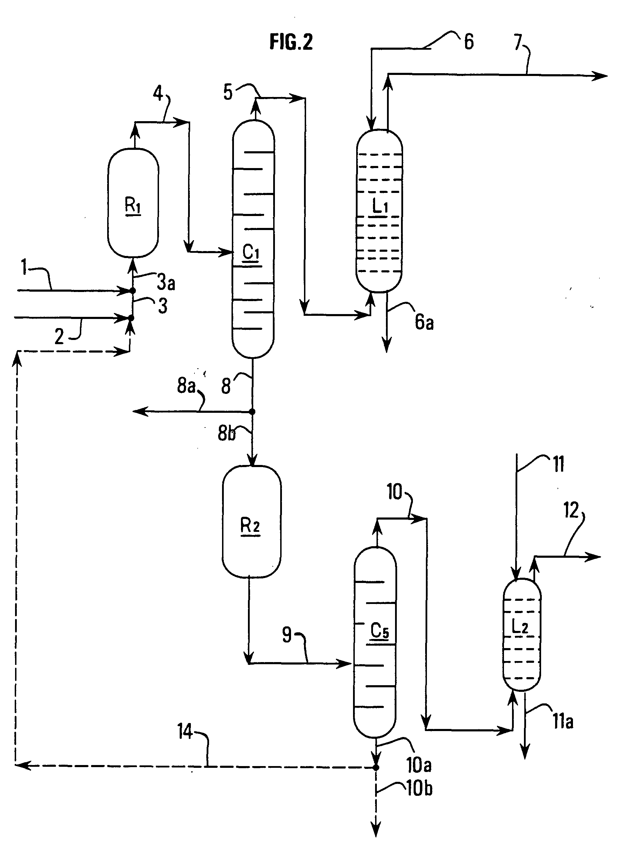

Selon une variante permettant d'améliorer encore la pureté des oléfines recherchées (voir en particulier la figure 2), le procédé de la présente invention comprend une étape e) dans laquelle au moins une partie de la fraction organique (O1) issue de la zone de fractionnement (C1) de l'étape b) est envoyée par la ligne 5 dans une zone d'extraction par lavage à l'eau (L1) dans laquelle l'eau nécessaire au lavage est introduite par la ligne 6. Dans cette étape e) et à partir de cette zone d'extraction (L1) par lavage à l'eau on obtient par la ligne 6a une fraction aqueuse (H1) contenant de l'eau et la majeure partie de l'alcool initialement présent dans ladite partie de produit (O1) et par la ligne 7 une fraction (O2) contenant la majeure partie de l'alpha oléfine initialement présente dans ladite partie de produit (O1), ladite fraction (O2) contenant ladite alpha oléfine purifiée (αhp1), de l'eau, et éventuellement d'autres composés hydrocarbonés provenant notamment de la coupe hydrocarbonée et étant sensiblement exempte d'alcool. La fraction (H1) peut être envoyée dans une section de traitement des eaux.

Dans le cadre du procédé de la présente invention (voir en particulier la figure 2) en complément ou séparément de la variante décrite ci-devant, le procédé selon l'invention comprendra une étape f) dans laquelle au moins une partie de la fraction organique (B1) issue de la zone de fractionnement (C5) de l'étape d) est envoyée par la ligne 10 dans une zone d'extraction par lavage à l'eau (L2) dans laquelle l'eau nécessaire au lavage est introduite par la ligne 11. Dans cette étape f) et à partir de cette zone d'extraction (L2) par lavage à l'eau on obtient par la ligne 11a une fraction aqueuse (H2) contenant de l'eau et la majeure partie de l'alcool initialement présent dans ladite partie de produit (B1) et par la ligne 12 une fraction (B2) contenant la majeure partie de l'oléfine tertiaire initialement présente dans ladite partie de produit (B1), ladite fraction (B2) contenant ladite oléfine tertiaire purifiée (Ohp1), de l'eau, et éventuellement des composés légers et étant sensiblement exempte d'alcool. La fraction (H2) peut être envoyée dans une section de traitement des eaux.

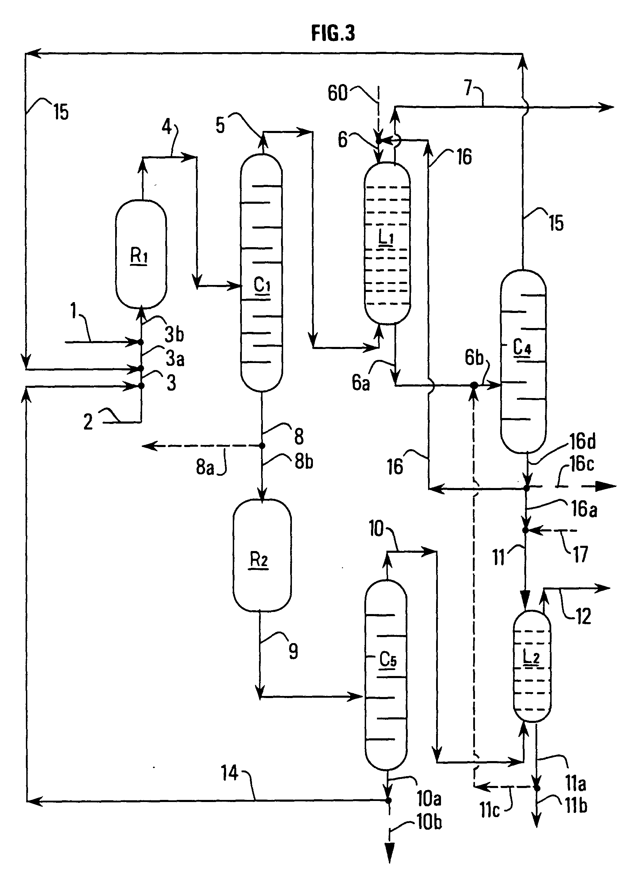

Selon une autre variante (voir en particulier la figure 3) dans le cas de l'utilisation d'une partie de la fraction (H1) obtenue à l'étape e), dans le cas de la présence d'au moins une des zones suivantes : une zone (L1) réalisant l'étape (e) et d'une zone (L2) réalisant l'étape (f), le procédé de la présente invention comprend une étape g) dans laquelle au moins une partie de la fraction aqueuse (H1) obtenue à l'étape e) (respectivement (H₂), obtenue à l'étape f)) d'extraction par lavage à l'eau à partir de la zone (L1) (respectivement L2) est envoyée par les lignes 6a et 6b (cas de la figure 3) dans une zone de fractionnement (C4) permettant d'obtenir d'une part une fraction aqueuse (H3) appauvrie en alcool que l'on récupère par la ligne 16d et que l'on envoie au moins en partie ou en totalité par les lignes 16d, 16 et 6 dans la zone d'extraction (L1) par lavage à l'eau de l'étape e) et/ou au moins en partie ou en totalité par les lignes 16d, 16a et 11 dans la zone éventuelle (L2) (présente sur la figure 3) d'extraction par lavage à l'eau de l'étape f) et d'autre part une fraction (A2) enrichie en alcool, que l'on peut au moins en partie renvoyer par les lignes 15, 3a et 3b dans la zone (R1) d'éthérification de l'étape a). Selon une variante il est aussi possible d'envoyer au moins une partie de la fraction aqueuse (H3) par les lignes 16d et 16c vers une zone de traitement des eaux. Dans cette réalisation, de l'eau d'appoint nécessaire au lavage peut être introduite dans la zone d'extraction (L1) par lavage à l'eau par les lignes 60 et 6 et de même de l'eau d'appoint nécessaire au lavage peut être introduite dans la zone éventuelle (L2) d'extraction par lavage à l'eau par les lignes 17 et 11. Dans cette réalisation il est également possible de recycler au moins une partie ou la totalité de la fraction aqueuse (H2), dans le cas de la présence d'une zone (C2) réalisant l'étape f), par les lignes 11a, 11c et 6b dans la zone de fractionnement (C4) de l'étape g) et/ou de récupérer au moins en partie ou en totalité cette fraction aqueuse (H2) par les lignes 11a et 11b que l'on peut envoyer dans une section de traitement des eaux.

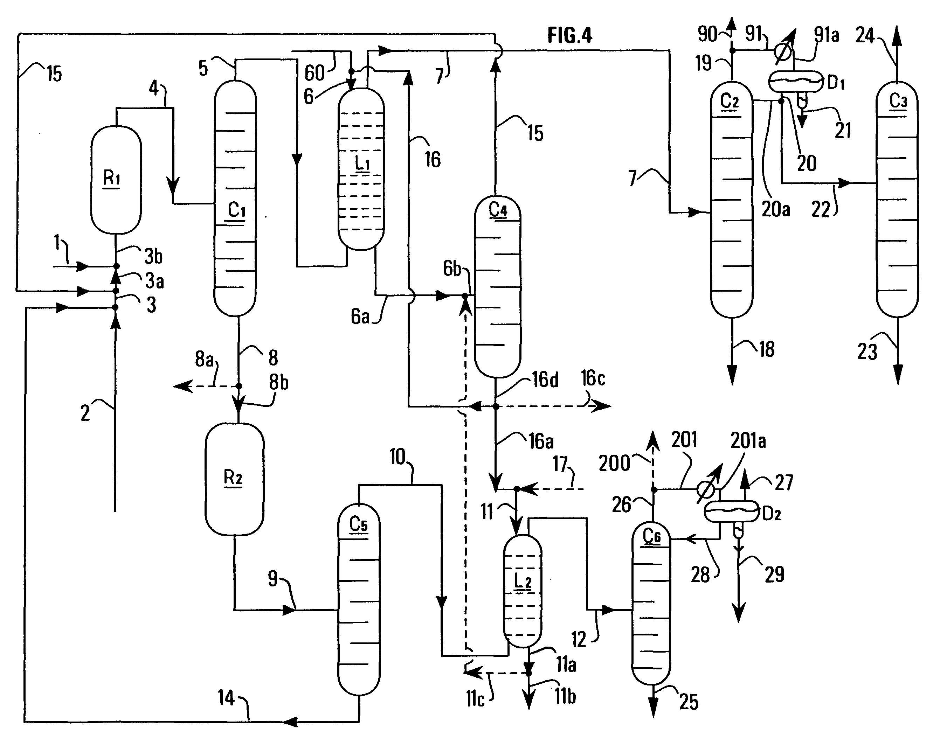

Selon une autre variante (voir en particulier la figure 4), dans le cas de la présence d'une zone (L1) réalisant l'étape e), le procédé de la présente invention comprend une étape h) dans laquelle au moins une partie de la fraction (O2) issue de la zone (L1) de l'étape e) contenant l'alpha oléfine purifiée (αhp1), de l'eau, et éventuellement des composés légers est envoyée par la ligne 7 dans une zone de fractionnement (C2), à partir de laquelle on récupère par les lignes 19 et 90 une fraction (O3) contenant de l'alpha oléfine de haute pureté (αhp2). A partir de ladite zone de fractionnement (C2) on obtient également par la ligne 18 une fraction (O5) appauvrie en alpha oléfine et contenant généralement notamment une ou plusieurs oléfines linéaires internes (par exemple du butène-2 dans le cas d'une coupe C4) et des composés linéaires habituellement saturés (par exemple du butane dans le cas d'une coupe C4). Il est possible d'envoyer au moins une partie et de préférence la totalité de la fraction (O3) par les lignes 19, 91 et 91a dans une zone de séparation D1 à partir de laquelle on récupère par la ligne 21 une fraction constituée en majeure partie d'eau, par la ligne 20 une fraction liquide essentiellement organique que l'on renvoie au moins en partie à titre de reflux dans la colonne C2 par la ligne 20a. Le plus souvent une partie au moins de ladite fraction liquide essentiellement organique qui contient de l'alpha oléfine de haute pureté (αhp2) est récupérée par les lignes 20 et 22. Dans cette réalisation, la ligne 91 puis 91a comporte un condenseur permettant de liquéfier au moins en partie et de préférence en totalité la fraction (O3). Dans le cas où cette fraction n'est pas liquéfiée en totalité la fraction gazeuse restante sera évacuée par une ligne non schématisée sur la figure 4 à partir de la zone de séparation (D1). Ladite fraction gazeuse est par exemple en partie envoyée à la torche. La fraction aqueuse récupérée par la ligne 21 peut être par exemple renvoyée en partie dans une zone d'extraction à l'eau du présent procédé.

Selon un autre des modes de réalisation de ladite variante (voir en particulier la figure 4) le procédé de la présente invention comprend en outre une étape i) dans laquelle au moins une partie de la fraction liquide essentiellement organique qui contient de l'alpha oléfine de haute pureté (αhp2) est envoyée par la ligne 22 dans une zone de fractionnement (C3) à partir de laquelle on obtient par la ligne 24 une fraction hydrocarbonée (O6) contenant notamment des composés saturés (paraffines) appauvrie en alpha oléfine et par la ligne 23 une fraction organique (04) contenant essentiellement de l'alpha oléfine ultra pure (αhp3).

Selon une autre variante (voir en particulier la figure 4), dans le cas de la présence d'une zone (L2) réalisant l'étape f), le procédé de la présente invention comprendra une étape j) dans laquelle au moins une partie de la fraction (B2) contenant l'oléfine tertiaire purifiée (Ohp1), de l'eau, et éventuellement des composés légers issue de la zone (L2) est envoyée par la ligne 12 dans une zone de fractionnement (C6) à partir de laquelle on récupère par la ligne 25 une fraction (B3) contenant de l'oléfine tertiaire de haute pureté (Ohp2). A partir de ladite zone de fractionnement on obtient également une fraction (B4) par les lignes 26 et 200 appauvrie en oléfine tertiaire contenant généralement notamment des composés légers (tels que par exemple du diméthyl éther, une faible quantité d'eau et un peu d'isobutène). Il est possible d'envoyer au moins une partie et de préférence la totalité de la fraction (B4) par les lignes 26, 201 et 201a dans une zone de séparation D2 à partir de laquelle on récupère par la ligne 29 une fraction constituée en majeure partie d'eau, par la ligne 28 une fraction liquide essentiellement organique que l'on renvoie au moins en partie à titre de reflux dans la colonne C6 par ladite ligne 28. Dans cette réalisation, la ligne 201 puis 201a comporte un condenseur permettant de liquéfier au moins en partie la fraction (B4). La fraction gazeuse est évacuée par la ligne 27 à partir de la zone de séparation (D2). Ladite fraction gazeuse est par exemple en partie envoyée à la torche. La fraction aqueuse récupérée par la ligne 29 peut être par exemple renvoyée en partie dans une zone d'extraction à l'eau du présent procédé.

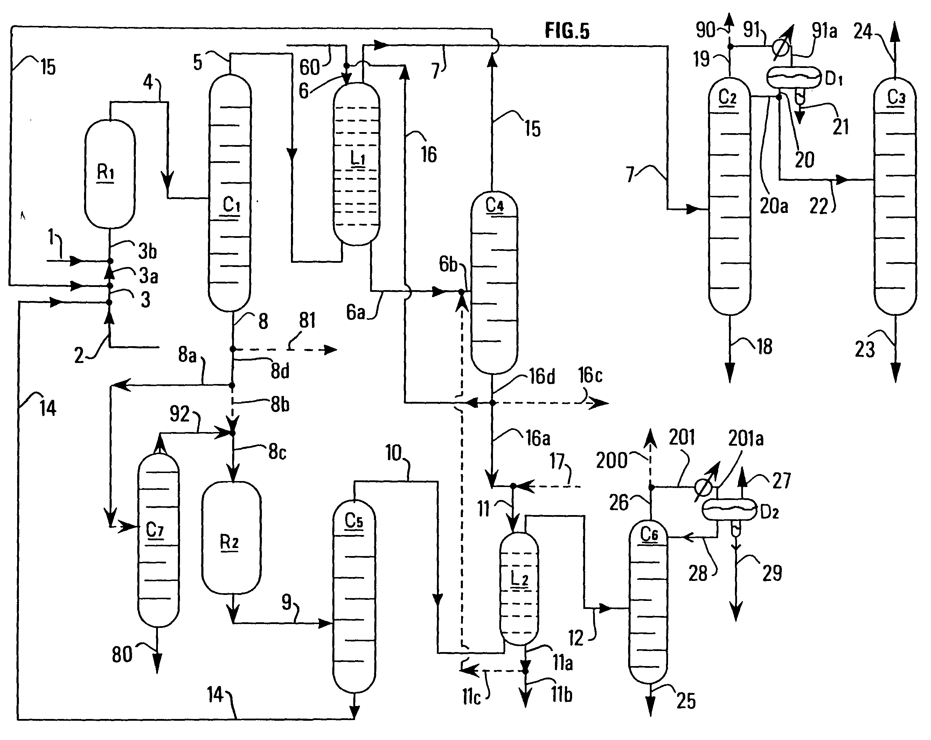

Selon une autre variante (voir en particulier la figure 5), le procédé de la présente invention comprend une étape k) dans laquelle au moins une partie de la fraction (E1) issue de la zone C1 réalisant l'étape b) est envoyée par les lignes 8, 8d et 8a dans une zone de fractionnement (C7) à partir de laquelle on récupère par la ligne 92 une fraction (E2) enrichie en éther alkylique tertiaire et appauvrie en composé lourds et par la ligne 80 une fraction (Q) appauvrie en éther alkylique tertiaire et enrichie en composés lourds (par exemple en dimères de l'isobutène et en MSBE dans le cas de traitement d'une coupe C4). Cette fraction (Q) peut être envoyée aux fractions carburant ou être éliminée par exemple à la torche. La fraction (E2) est habituellement au moins en partie envoyée par les lignes 92 et 8c dans la zone (R2) de décomposition de l'éther alkylique tertiaire de l'étape c). Lorsque une partie seulement de la fraction (E1) est envoyée à l'étape k), l'autre partie peut être introduite par les lignes 8b et 8c, de préférence en mélange avec au moins une partie de la fraction (E2), dans la zone de décomposition de l'éther de l'étape c). Selon une variante une partie de la fraction (E1) peut aussi être envoyée par les lignes 8 et 81 aux fractions carburant. Selon la présente variante il est également possible de récupérer au moins une partie de la fraction (E2) contenant de l'éther alkylique tertiaire purifié qui peut être éventuellement envoyée aux fractions ou être utilisée à d'autres usages.

Les conditions de mise en oeuvre de l'étape a) de la présente invention sont des conditions classiques bien connues de l'homme du métier pour la synthèse d'éther alkylique tertiaire à partir de coupe hydrocarbonée contenant au moins une oléfine tertiaire éthérifiable par au moins un alcool, en particulier un alcool ayant de 1 à 6 atomes de carbone par molécule et le plus souvent le méthanol ou l'éthanol. La synthèse d'éther est le plus souvent effectuée en présence d'un catalyseur acide et le plus souvent d'un catalyseur acide solide choisi dans le groupe formé par les résines acides organiques (par exemple des résines sulfoniques) et les résines acides minérales, généralement solides dans les conditions de la réaction de synthèse d'éther (par exemple les solides minéraux greffés comportant au moins un groupe organique sulfonique par exemple alkyl-sulfonique, arylsulfonique, alkylaryl-sulfonique et en particulier les polysiloxanes greffés et plus particulièrement ceux greffés par au moins un groupe alkyl-sulfonique). Ledit catalyseur peut être une résine commerciale telle que la résine Amberlyst 15 ou 35 de la société ROHM et HAAS ou la résine M 31 de la société DOW-CHEMICAL, ou un polysiloxane greffé commercial. Dans cette étape a) la quantité d'alcool employée est habituellement telle que le rapport molaire alcool/oléfine tertiaire présente dans la charge est d'environ 0,5 : 1 environ 8 : 1, souvent d'environ 0,8 : 1 environ 5 : 1, le plus souvent d'environ 0,9 : 1 à environ 4 : 1. La température de réaction est habituellement d'environ 20 °C à environ 120 °C, souvent d'environ 30 °C à environ 100 °C, le plus souvent d'environ 40°C à environ 90 °C. La VVH (vitesse volumique horaire) en volume de charge par volume de catalyseur et par heure est habituellement d'environ 0,005 à environ 100, souvent d'environ 0,01 à environ 50 et le plus souvent d'environ 0,1 à environ 10. La pression est habituellement choisie de manière à ce que les constituants présents dans la zone de réaction soient à l'état liquide. Habituellement la pression absolue dans cette zone d'éthérification est d'environ 1 bar à environ 40 bar, souvent d'environ 1 bar à environ 25 bar (1 bar est égal à 0,1 MPa).

Les conditions de mise en oeuvre de l'étape b) de séparation du produit issu de l'étape a) en une fraction organique (O1) ne contenant pratiquement pas d'éther alkylique tertiaire et une fraction organique (E1) de préférence contenant pratiquement la totalité de l'éther alkylique tertiaire formé au cours de l'étape a) sont des conditions classiques qui dépendent des composés présents dans le produit issu de l'étape a). Cette séparation peut être effectuée dans des conditions plus ou moins sévères permettant d'obtenir une fraction (O1) contenant encore une faible proportion d'éther. L'homme du métier est à même de choisir les conditions opératoires en vue d'obtenir la séparation souhaitée. Les conditions sont le plus souvent choisies de manière à obtenir une fraction (E1) contenant la quasi totalité de l'éther formé au cours de l'étape a). Dans le cadre de l'invention la zone réactionnelle d'éthérification de l'étape a) peut être distincte de la zone de séparation ou fractionnement de l'étape b), ou bien on peut employer un appareillage comprenant une zone mixte de réaction et de fractionnement (colonne de distillation catalytique) comme cela a par exemple été décrit dans de nombreux brevets et publications de l'art antérieur. Il est souvent souhaitable que la zone d'éthérification soit composée d'une section réactionnelle principale et d'au moins une section réactionnelle de finition, cette dernière comprenant de préférence soit un réacteur contenant un lit fixe de catalyseur, soit une colonne réactive (colonne de distillation-réaction), soit la combinaison des deux. Le procédé de la présente invention peut comporter l'utilisation d'une zone de distillation réactive regroupant les fonctions de l'étape a) et celles de l'étape b), c'est-à-dire effectuant simulténément l'étape a) et l'étape b) du procédé selon l'invention, soit utilisant pour effectuer ces étapes un appareillage comprenant au moins une colonne de distillation réactive.

Dans le cas d'un procédé dans lequel on fabrique dans l'étape a) du MTBE, la zone de séparation par distillation est généralement une colonne travaillant habituellement sous une pression absolue d'environ 1 à environ 30 bar, identique ou différente de celle régnant dans la zone (R1) d'éthérification. Ladite colonne comporte habituellement de 3 à 80 plateaux théoriques et souvent de 10 à 50 plateaux théoriques. Une forme préférée de réalisation de l'invention comprend une zone d'éthérification comprenant au moins un réacteur (R1) d'éthérification et une zone de fractionnement comportant une colonne de distillation catalytique contenant un catalyseur d'éthérification dans laquelle l'éthérification est poursuivie tout en effectuant la distillation permettant la séparation d'une fraction éther et d'une fraction alpha oléfine.

Les conditions de mise en oeuvre de l'étape c) de la présente invention sont des conditions classiques de décomposition d'éther alkylique tertiaire bien connues de l'homme du métier. Dans une forme préférée de réalisation de cette étape c) sera mise en oeuvre sans addition d'eau supplémentaire au produit introduit dans la zone de décomposition. Il serait cependant possible d'ajouter une certaine quantité d'eau par exemple jusqu'à la limite de solubilité de l'eau dans l'éther que l'on souhaite décomposer. Habituellement les conditions de mise en oeuvre de l'étape c) dans la zone (R2) sont choisies de manière à ce que la majeure partie de l'éther alkylique tertiaire se décompose pour donner un alcool et une oléfine tertiaire. Dans ladite zone (R2) la pression absolue est habituellement d'environ 1 à environ 30 bar, de préférence d'environ 1 à environ 12 bar, la température est habituellement comprise entre 50 °C et 300 °C et de préférence entre 100 °C et 250°C, et la VVH (vitesse spatiale horaire) dépend du catalyseur utilisé. Dans ladite zone (R2), on peut utiliser tous les catalyseurs acides bien connus de l'homme du métier. On préfère habituellement employer des catalyseurs solides acides . Ainsi le catalyseur peut être choisi dans le groupe formé par les résines acides organiques et les résines acides minérales généralement solides dans les conditions de la réaction de décomposition dudit éther. Parmi ces composés on emploie le plus souvent ceux choisis dans le groupe formé par les solides minéraux greffés comportant au moins un groupe organique de type alkyl-sulfonique, aryl-sulfonique ou alkylaryl-sulfonique. L'une des formes préférée de mise en oeuvre de cette étape c) utilise un catalyseur choisi dans le groupe formé par les polysiloxanes greffés par au moins un groupe alkyl-sulfonique.

Les conditions générales de mise en oeuvre de l'étape d) de fractionnement du produit (P1) issu de l'étape c), dans la zone de fractionnement (C5) sont choisies en particulier en fonction des caractéristiques de l'alcool et de l'oléfine tertiaire formés. L'homme du métier est à même de choisir ces conditions pour obtenir la séparation souhaitée entre une fraction contenant la majeure partie de l'alcool et une fraction contenant la majeure partie de l'oléfine. Ainsi par exemple dans le cas de la décomposition du MTBE et de la formation de méthanol et d'isobutène la pression absolue dans la zone (C5) qui est une colonne de distillation est d'environ 1 à environ 15 bar, de préférence d'environ 1 à environ 10 bar, identique ou différente de celle régnant dans la zone (R2) de décomposition. La température de fond de la colonne dépend à la fois de la pression régnant dans ladite colonne et de la composition du produit de fond, en particulier du rapport molaire entre le méthanol et le MTBE éventuellement présent par suite d'une décomposition partielle de cet éther dans l'étape c). Dans le cas d'une unité traitant 1 kg/h de MTBE la colonne de distillation comporte habituellement entre 3 et 80 plateaux théoriques et le plus souvent entre 10 et 50 plateaux théoriques.

Dans l'étape éventuelle e) de purification, au moins une partie de la fraction (O1), contenant la majeure partie de l'alpha oléfine obtenue à l'étape b), est envoyée dans une zone d'extraction (L1) par lavage à l'eau. La quantité d'eau utilisée pour ce lavage est habituellement telle que le rapport volumique entre le volume de ladite quantité d'eau introduite dans ladite zone d'extraction et celui de ladite partie de fraction (O1) introduite dans ladite zone d'extraction (Vₑₐᵤ/V_{O1}) est d'environ 0,005 à environ 20. Le plus souvent cette quantité d'eau est telle que le rapport Vₑₐᵤ/V_{O1} soit d'environ 0,005 à environ 10, de préférence d'environ 0,01 à environ 5 et de manière encore plus préférée d'environ 0,02 à environ 1. Le débit d'eau dans cette zone de lavage (L1) est très souvent régulé en fonction du maintien d'un niveau de fond de l'eau et de l'alcool dans la zone de fractionnement (C4) lors de la présence d'une telle zone (C4) réalisant l'étape g). Ledit niveau de fond peut être défini comme le niveau minimal nécessaire au bon fonctionnement de ladite zone de fractionnement C4. Ce paramètre est un paramètre classique bien connu de l'homme du métier. Cette régulation est souvent faite en mode manuel par les opérateurs, mais il est possible que cette régulation soit effectuée par une boucle de régulation automatique dite LCR des initiales anglo-saxonnes Level Control Regulation. Quel que soit le mode de régulation choisi, la quantité d'eau est généralement ajustée à l'aide d'un moyen d'introduction d'eau d'appoint dans ladite zone (L1) de lavage à l'eau de l'étape e). Cet appoint d'eau permet en particulier de compenser les pertes d'eau dues à l'entraînement d'eau et/ou à la saturation du flux hydrocarboné traité, ainsi que le remplacement de l'eau éventuellement purgée. Cette zone (L1) d'extraction est habituellement une colonne à plateaux qui opère à une température d'environ 1 à environ 100 °C, de préférence d'environ 10 à environ 60 °C. La pression absolue dans cette zone est d'environ 1 à environ 20 bar, le plus souvent d'environ 1 à environ 15 bar, identique ou différente de celle régnant dans la zone de fractionnement (C1) de l'étape b).

Dans l'étape f) éventuelle de purification, au moins une partie de la fraction (B1), contenant la majeure partie de l'oléfine tertiaire obtenue à l'étape d), est envoyée dans une zone (L2) d'extraction par lavage à l'eau. La quantité d'eau utilisée pour ce lavage est habituellement telle que le rapport volumique entre le volume de ladite quantité d'eau introduite dans ladite zone d'extraction et celui de ladite partie de fraction (B1) introduite dans ladite zone d'extraction (Vₑₐᵤ/V_{B1}) est d'environ 0,005 à environ 20. Le plus souvent, cette quantité d'eau est telle que le rapport Vₑₐᵤ/V_{B1} est d'environ 0,005 à environ 10, de préférence d'environ 0,01 à environ 5 et de manière encore plus préférée d'environ 0,02 à environ 1. Le débit d'eau dans cette zone de lavage (L1) est très souvent régulé en fonction du maintien d'un niveau de fond de l'eau et de l'alcool dans la zone de fractionnement (C6) lors de la présence d'une telle zone de fractionnement (C6) réalisant l'étape g). Ce niveau de fond peut être défini comme le niveau minimal nécessaire au bon fonctionnement de la colonne de fractionnement C6. Ce paramètre est un paramètre classique bien connu de l'homme du métier. Cette régulation est souvent faite en mode manuel par les opérateurs, mais il est possible que cette régulation soit effectuée par une boucle de régulation automatique dite LCR des initiales anglo-saxonnes Level Control Regulation. Quel que soit le mode de régulation choisi, la quantité d'eau est généralement ajustée à l'aide d'un moyen d'introduction d'eau d'appoint dans ladite zone (L2) de lavage à l'eau de l'étape f). Cet appoint d'eau permet en particulier de compenser les pertes d'eau dues à l'entraînement d'eau et/ou à la saturation du flux hydrocarboné traité ainsi que le remplacement de l'eau éventuellement purgée. Ladite zone (L2) d'extraction est habituellement une colonne à plateaux qui opère à une température d'environ 1 à environ 100 °C, de préférence d'environ 10 à environ 60 °C. La pression absolue dans cette zone est d'environ 1 à environ 20 bar, le plus souvent d'environ 1 à environ 15 bar, identique ou différente de celle régnant dans la zone de fractionnement (C5) de l'étape d).

L'étape g) éventuelle de fractionnement, dans le cas de la présence d'une zone (L1) réalisant l'étape e) et/ou de la présence d'une zone (L2) réalisant l'étape f), d'au moins une partie de la (ou des) fraction(s) aqueuse(s) (H1) et/ou (H2), contenant la majeure partie de l'alcool initialement présent dans la fraction (et/ou dans le mélange des fractions) (H1) récupérée de la zone (L1) éventuelle à l'étape e) d'extraction à l'eau et/ou (H2) récupérée de la zone (L2) éventuelle à l'étape f) d'extraction à l'eau dans laquelle on sépare au moins une partie desdit mélange de fraction ou ladite fraction (H1) et/ou (H2) en une fraction (A2) contenant la majeure partie de l'alcool initialement présent dans ladite partie de mélange ou de ladite fraction (H1) et/ou (H2) et en une fraction aqueuse (H3) débarrassée de la majeure partie de l'alcool initialement présent dans ladite partie dudit mélange de fraction ou ladite fraction (H1) et/ou (H2), est habituellement réalisée dans une zone de fractionnement (C4) qui est généralement une colonne à distiller (C4) sous une pression absolue d'environ 1 à environ 12 bar, de préférence d'environ 1 à environ 8 bar, identique ou différente de celle régnant dans les zones (L1) et/ou (L2) d'extractions à l'eau de l'étape e) et de l'étape f). La température de fond de la colonne dépend en particulier de la pression régnant dans ladite colonne, elle est habituellement d'environ 50 à environ 300 °C et le plus souvent d'environ 65 à environ 200°C. La colonne comporte habituellement environ 2 à environ 80 plateaux et le plus souvent environ 3 à environ 60 plateaux théoriques.

L'étape h) éventuelle de fractionnement d'au moins une partie de la fraction (02) issue de la zone (L1), dans le cas de la présence d'une telle zone (L1) réalisant l'étape e), en une fraction (O3) contenant l'oléfine tertiaire purifiée (αhp2), l'eau résiduelle contenue dans la fraction liquide (O2) et des composés légers et en une fraction (05) contenant la majeure partie des composés saturés et des oléfines internes linéaires éventuellement présents dans ladite partie de fraction (O2) est habituellement effectuée dans une zone de fractionnement (C2) qui est généralement une colonne à distiller fonctionnant sous une pression absolue d'environ 1 à environ 15 bar, le plus souvent d'environ 3 à environ 10 bar, identique ou différente de celle régnant dans la zone d'extraction (L1) de l'étape e). Pour une unité produisant 0,3 kg/h de butène-1, ladite colonne aura habituellement environ 3 à environ 100 plateaux théoriques et le plus souvent environ 10 à environ 70 plateaux théoriques. La température de fond de la colonne dépend en particulier de la pression régnant dans ladite colonne.

L'étape i) éventuelle de fractionnement d'au moins une partie de la fraction (O3) issue de la zone (C2), dans le cas de la présence d'une zone (L1) réalisant l'étape e) et d'une zone (C2) réalisant l'étape h), en une fraction (O4) contenant l'oléfine tertiaire purifiée (αhp3) et en une fraction (06) contenant la majeure partie des composés saturés éventuellement présents dans ladite partie de fraction (03), est habituellement effectuée dans une colonne à distiller fonctionnant sous une pression absolue d'environ 1 à environ 15 bar, le plus souvent d'environ 3 à environ 10 bar, identique ou différente de celle régnant dans la zone de fractionnement (C2) de l'étape h). Pour une unité produisant 0,3 kg/h de butène-1 cette colonne aura habituellement environ 3 à environ 80 plateaux théoriques et le plus souvent environ 5 à environ 50 plateaux théoriques. La température de fond de la colonne dépend en particulier de la pression régnant dans ladite colonne.

L'étape j) éventuelle de fractionnement d'au moins une partie de la fraction (B2) issue de la zone (L2), dans le cas de la présence d'une zone (L2) réalisant l'étape f), en une fraction (B3) contenant l'oléfine tertiaire purifiée (Ohp2) et en une fraction (B4) contenant la majeure partie des composés légers éventuellement présents dans ladite fraction (B2) et l'eau résiduelle contenue dans ladite partie de fraction liquide (B2), est habituellement effectuée dans une zone de fractionnement (C6) qui est généralement une colonne à distiller fonctionnant sous une pression absolue d'environ 1 à environ 15 bar, le plus souvent d'environ 3 à environ 10 bar, identique ou différente de celle régnant dans la zone (L2) d'extraction de l'étape f). Pour une unité produisant 0,6 kg/h d'isobutène, ladite colonne aura habituellement environ 3 à environ 80 plateaux théoriques et le plus souvent environ 5 à environ 50 plateaux théoriques. La température de fond de la colonne dépend en particulier de la pression régnant dans ladite colonne.

Selon une forme particulière permettant d'améliorer encore le procédé de la présente invention, au moins une partie de la ou les fraction(s) organique(s) contenant (1') les oléfine(s) recherchée(s) obtenue(s) à la sortie d'au moins une zone (L1) ou (L2) d'extraction par lavage à l'eau dans le cas de la présence d'au moins une telle zone (L1) ou (L2), qui est associée à une zone (C2), la zone (L1) étant associée à la zone (C2) réalisant l'étape h) selon l'invention et la zone (L2) étant associée à la zone C6) réalisant l'étape j) selon l'invention, est (sont) envoyée(s) dans une zone de séparation (Coe) à partir de laquelle on récupère une fraction liquide aqueuse (Lae) et une fraction liquide organique (Lbe) contenant la majeure partie de l'oléfine initialement présente dans ladite partie; ladite fraction (Lbe) appauvrie en eau et qui contient ladite oléfine et éventuellement d'autres composés, est alors envoyée respectivement dans la zone de fractionnement (C2) dans le cas de la présence d'une zone (L1)et (C6) dans le cas de la présence d'une zone (L2) à partir desquelles on obtient respectivement de l'alpha oléfine purifiée (α'hp2) et de l'oléfine tertiaire purifiée (O'hp2). Cette réalisation qui comprend une zone de séparation d'une fraction liquide aqueuse (Lae) et d'une fraction liquide organique (Lbe) est habituellement mise en oeuvre dans un appareil dénommé coalesceur, dans lequel l'eau se rassemble à la partie inférieure de l'appareil par coalescence. Les conditions de température et de pression régnant dans cette zone sont dans les mêmes gammes que celles régnant dans l'étape correspondante et précédente d'extraction à l'eau. La pression respectivement la température peut être identique ou différente de celle régnant dans ladite étape précédente. Dans ladite zone (coe) on sépare ainsi l'eau libre contenue dans le produit issu de l'étape d'extraction à l'eau. De plus cette zone a également le plus souvent une fonction de zone ou ballon de charge pour la zone ultérieure de purification de l'oléfine. Tout autre moyen connu peut être employé dans le cadre de la présente invention, telle que l'utilisation d'un absorbant ayant une sélectivité préférentielle pour l'une des fractions aqueuse ou organique.

L'exemple suivant illustre l'invention sans en limiter la portée.

### Exemple

On utilise un équipement de type pilote comprenant trois réacteurs tubulaires (R1) (R2) et (R3) pour réaliser les étapes réactionnelles de synthèse et de décomposition de l'éther. Le premier réacteur (R1) de 20 millilitres de volume contient 6 grammes de résine Amberlyst 15 vendue par la société ROHM ET HAAS et fonctionne sous une pression relative de 10 bar, à une température moyenne de 50 °C. Il est alimenté par une coupe C₄ issue du craquage catalytique, contenant 20 % en poids d'isobutène et 20 % en poids de butène-1, et par du méthanol pur vendu par la société ALDRICH comme un produit ayant une pureté supérieure à 99 % en poids. La quantité de méthanol est ajustée de manière à ce que le rapport molaire méthanol/isobutène introduit dans le réacteur (R1) soit de 1,2. Le réacteur travaille avec une VVH de 0,5 h⁻¹. Dans les conditions choisies la conversion de l'isobutène en MTBE est égale à 97 %. L'effluent de R1 est repris dans une seconde étape d'éthérification, comprenant le réacteur R2 de caractéristiques identiques à celles du réacteur R1 et contenant le même catalyseur en quantité identique, fonctionnant dans les mêmes conditions que R1. 95 % de l'isobutène non converti dans R1 est alors transformé en MTBE dans R2. Sur l'ensemble R1 et R2, on obtient alors une conversion globale de l'isobutène en MTBE de 99,8 %.

Le troisième réacteur (R3) de volume égal à 10 millilitres contient 3 grammes d'un catalyseur commercial de type polysiloxane greffé par au moins un groupement alkyl sulfonique. On alimente le réacteur R3 par une charge contenant 100 % poids de MTBE, sous une VVH de 15 h⁻¹, la pression relative dans le réacteur est de 7 bar et la température moyenne de étant de 160 °C. Le tableau 1 présente la composition de la charge introduite dans le réacteur R3 de décomposition du MTBE et la composition du produit recueilli à la sortie du réacteur R3.

**Tableau 1**

| | Charge(% poids) | Effluent R3 (% poids) |
|---|---|---|
| MTBE | 100 | 10 |
| Isobutène | | 56,1 |
| Méthanol | | 32,1 |
| DME | | 0,5 |
| Dimères | | 1,1 |
| H2O | | 0,2 |

A l'aide du logiciel commercialisé par la société américaine SIMSCI (SIMulation SCIence INC.) sous la dénomination commerciale Pro II, on calcule les diverses sections de purification.

Une colonne de distillation (C1), fonctionnant sous une pression relative de 7 bar, comportant 10 plateaux théoriques est utilisée à l'étape b) du procédé de l'invention pour obtenir un produit de fond (E1) et un produit de tête (O1), (On simule ici le fractionnement du produit issu de l'étape a) d'éthérification de l'isobutène contenu dans la coupe C4).

Une colonne de distillation (C5), fonctionnant sous une pression relative de 7 bar, comportant 30 plateaux théoriques est utilisée à l'étape d) du procédé de l'invention pour obtenir un produit de fond (A1) et un produit de tête (B1), (On simule ici le fractionnement isobutène/méthanol issu de l'étape c) de décomposition du MTBE).

Une colonne d'extraction par lavage à l'eau (L2), qui est une colonne à plateaux et qui fonctionne à température 30 °C, est utilisée dans l'étape f) du procédé de l'invention pour obtenir une fraction aqueuse (H2) et une fraction organique (B2) (on simule ici l'extraction du méthanol résiduel à partir de la fraction (B1) issu de l'étape d) de fractionnement du produit issu de l'étape c) de décomposition du MTBE).

Un système d'extraction de l'eau libre entraînée est utiliséà l'étape f) dans la fraction (B2), du type coalesceur (Co2) à partir duquel on obtient une fraction aqueuse (La2) et une fraction organique (Lb2) qui fonctionne sous une pression relative de 12 bar à une température de 30 °C.

Une colonne de distillation (C6), dernière étape de purification de l'isobutène qui fonctionne sous une pression relative de 7 bar, comportant 25 plateaux théoriques est utilisée à l'étape j) du procédé de l'invention pour obtenir un produit de fond (B3) qui est de l'isobutène purifié (Ohp3) et un produit de tête (B4) contenant des composés légers.

Une colonne d'extraction par lavage à l'eau (L1), qui est une colonne à plateaux et qui fonctionne à température 30 °C, est utilisée dans l'étape e) du procédé de l'invention pour obtenir une fraction aqueuse (H1) et une fraction organique (O2) (on simule ici l'extraction du méthanol résiduel à partir de la fraction (O1) issu de l'étape b) de fractionnement du produit issu de l'étape a) d'éthérification).

Un système d'extraction de l'eau libre entraînée est utilisé à l'étape e) dans la fraction (O2), du type coalesceur (Co1) à partir duquel on obtient une fraction aqueuse (La1) et une fraction organique (Lb1) qui fonctionne sous une pression relative de 12 bar à une température de 30 °C.

Une colonne de distillation (C2) de purification de la fraction organique (Lb1) qui fonctionne sous une pression relative de 7 bar, comportant 20 plateaux théoriques est utilisée à l'étape h) du procédé de l'invention pour obtenir un produit de tête (O3) qui est du butène-1 purifié (αhp2) et un produit de fond (O5) contenant des composés en C4 hors l'isobutène et le butène-1.

Une colonne de distillation (C3) de purification de la fraction organique (O3) qui fonctionne sous une pression relative de 7 bar, comportant 20 plateaux théoriques est utilisée à l'étape i) du procédé de l'invention pour obtenir un produit de tête (O6) contenant de l'isobutane et un produit de fond (O4) qui est du butène-1 de très haute pureté (αhp3).

La colonne (C1) est alimenté par le produit issu du réacteur d'éthérification (R2). Le produit (E1) récupéré en fond de colonne (C1) est utilisé pour alimenter le réacteur (R3). Le produit (O1) recueilli en tête de C1 est envoyé dans une zone d'extraction par lavage à l'eau (L1) à partir de laquelle on obtient un produit (O2) contenant du butène-1 purifié (αhp1). La colonne C5 est alimentée par l'effluent P1 de R3. Le produit B1 recueilli en tête de C5 est envoyé dans la colonne d'extraction L2 où il est lavé par une certaine quantité d'eau. On recueille une fraction aqueuse H2 contenant la majeure partie du méthanol contenu dans B1, et une fraction hydrocarbonée B2 contenant une petite quantité d'eau libre entraînée. Cette fraction d'eau libre est enfin éliminée après décantation dans le système de décantation Co2. Enfin, la fraction hydrocarbonée Lb2 est traitée dans la colonne C6 afin de produire en fond une fraction B3 formée essentiellement d'isobutène de haute pureté (Ohp3) et en tête une fraction légère B4 contenant en particulier le diméthyl éther (DME).

Les bilans matières obtenus sont donnés dans les tableaux 2, 3, 4 et 5 ci-après. Le tableau 2 présente le bilan matière relatif aux deux premières étapes réactionnelles de synthèse du MTBE. Les tableaux 3 et 4 présentent le bilan matière relatif à la décomposition du MTBE et à la production d'isobutène de haute pureté. Le tableau 5 présente le bilan matière relatif à la purification du butène-1.

**Tableau 2**

| | Charge R1 (poids) | Effluent R1 (poids) | Effluent R2 (poids) | Tête C1 (poids) | Fond C1 (poids) |
|---|---|---|---|---|---|
| Isobutène | 20 | 0,6 | 0,03 | 0,03 | |
| Butène-1 | 20 | 20 | 20 | 20 | |
| C4 hors isobutène | 60 | 60 | 60 | 60 | |
| Méthanol | 13,7 | 2,7 | 2,37 | 2,37 | |
| MTBE | 0 | 30,4 | 31,3 | 0 | 31,3 |
| Total | 113,7 | 113,7 | 113,7 | 82,4 | 31,3 |

**Tableau 3**

| | Effluent R3 | Fond C5 fraction A1 (g/h) | Tête C5 fraction B1 (g/h) | Eau de lavage colonne L2 | Fraction aqueuse H2 colonne L2 | Fraction organique B2 colonne L2 |
|---|---|---|---|---|---|---|
| MTBE | 10 | 10 | | | | |
| Isobutène | 56,1 | | 56,1 | | | 56,1 |
| Méthanol | 32,1 | 30,4 | 1,7 | | 1,7 | |
| DME | 0,5 | | 0,5 | | | 0,5 |
| Dimères | 1,1 | 1,1 | | | | |
| H2O | 0,2 | | 0,2 | 10 | 9,2 | 1 |
| Débit (g/h) | 100 | 41,5 | 58,5 | 10 | 10,9 | 57,6 |

**Tableau 4**

| | Fraction aqueuse La2 extraite par Co2 | Fraction organique Lb2 extraite par Co2 | Fraction B4 colonne C6 | Fraction B3 colonne C6 |
|---|---|---|---|---|
| Isobutène | | 56,1 | 1,72 | 54,38 |
| DME | | 0,5 | 0,49 | 0,01 |
| H2O | 0,99 | 0,01 | 0,01 | |
| Débit (g/h) | 0,99 | 56,61 | 2,22 | 54,39 |
| Pureté de l'isobutène (%) | | | | 99,9 % |

**Tableau 5**

| | Tête C1 (poids) | Fraction O2 colonne L1 | Fraction O3 colonne C2 (poids) | Fraction O5 colonne C2 (poids) | Fraction O6 colonne C3 (poids) | Fraction O4 colonne C3 (poids) |
|---|---|---|---|---|---|---|
| Isobutène | 0,03 | 0,03 | 0,03 | | | 0,03 |
| Butène-1 | 20 | 20 | 20 | | | 20 |
| Isobutane | 15 | 15 | 15 | | 15 | |
| n-butane butène-2 | 45 | 45 | | 45 | | |
| Méthanol | 2,37 | | | | | |
| Total | 82,4 | 80,03 | 35,03 | 45 | 15 | 20,03 |

Cet exemple montre que la réalisation du procédé selon l'invention permet d'obtenir à partir d'une coupe C₄ contenant du butène-1 et de l'isobutène, un isobutène ayant une pureté de 99,9 %, avec une conversion globale de 83 % sans optimisation c'est-à-dire si l'on ne fait aucun recyclage, ainsi que du butène-1 ayant une pureté de 99,85 %. Le rendement en isobutène de haute pureté peut être amélioré par l'utilisation de divers recyclages dont celui de la fraction A1 ( fond de la colonne C5).

## Revendications

1. Procédé de production d'alpha oléfine pure, d'oléfine tertiaire pure et éventuellement d'éther alkylique tertiaire à partir de coupe hydrocarbonée insaturée contenant au moins une alpha oléfine et au moins une oléfine tertiaire éthérifiable, ledit procédé étant caractérisé en ce qu'il comprend :
a) une étape de formation d'au moins un éther alkylique tertiaire par mise en contact dans une zone réactionnelle, (R1) contenant un catalyseur d'éthérification, d'une coupe hydrocarbonée contenant au moins une alpha oléfine et au moins une oléfine tertiaire éthérifiable avec au moins un alcool,
b) une étape de fractionnement d'au moins une partie du produit issu de l'étape a) dans une zone de fractionnement (C1) permettant d'obtenir d'une part une fraction organique (O1) appauvrie en éther alkylique tertiaire et contenant l'alpha oléfine, et en une fraction organique (E1) enrichie en éther alkylique tertiaire formé au cours de l'étape a), ladite fraction organique (O1) étant ultérieurement purifiée par lavage à l'eau puis par fractionnement,
c) une étape de décomposition, d'au moins une partie de l'éther alkylique tertiaire contenu dans la fraction organique (E1) de l'étape b), dans une zone réactionnelle (R2) contenant un catalyseur de décomposition dudit éther en un produit (P1) contenant au moins un alcool et au moins une oléfine tertiaire, et
d) une étape de fractionnement d'au moins une partie du produit (P1) issu de l'étape c), dans une zone de fractionnement (C5) permettant d'obtenir d'une part une fraction (B1) contenant la majeure partie de l'oléfine tertiaire et éventuellement une fraction mineure d'alcool et éventuellement des composés légers éventuellement contenus initialement dans ladite partie de produit (P1), et d'autre part une fraction (A1) contenant la majeure partie de l'alcool formé dans l'étape c), éventuellement de l'éther non décomposé dans l'étape c) et éventuellement des composés lourds contenus initialement dans ladite partie de produit (P1) issu de l'étape c), ladite fraction (B1) étant ultérieurement purifiée par lavage à l'eau puis par fractionnement.

2. Procédé selon la revendication 1 dans lequel au moins une partie de la fraction (A1) est envoyée dans la zone (R1).

3. Procédé selon la revendication 1 ou 2 comprenant une étape e) dans laquelle au moins une partie de la fraction organique (O1) issue de la zone de fractionnement (C1) de l'étape b), est envoyée dans une zone (L1) d'extraction par lavage à l'eau à partir de laquelle on obtient une fraction aqueuse (H1) contenant de l'eau et la majeure partie de l'alcool initialement présent dans ladite partie de produit (O1) et une fraction (O2) contenant la majeure partie de l'alpha oléfine initialement présente dans ladite partie de produit (O1), ladite fraction (O2) contenant ladite alpha oléfine purifiée (ahpl), de l'eau, et éventuellement d'autres composés hydrocarbonés et étant sensiblement exempte d'alcool.

4. Procédé selon l'une des revendications 1 à 3 comprenant une étape f) dans laquelle au moins une partie de la fraction (B1) est envoyée dans une zone (L2) de lavage à l'eau à partir de laquelle on récupère une fraction (B2) contenant la majeure partie de l'oléfine tertiaire initialement présente dans ladite partie de produit (B1), ladite fraction (B2) contenant ladite oléfine tertiaire purifiée (Ohp1), de l'eau, et éventuellement des composés légers et étant sensiblement exempte d'alcool, et une fraction aqueuse (H2) contenant de l'eau et la majeure partie de l'alcool initialement présent dans ladite fraction de produit (B1).

5. Procédé selon l'une des revendications 3 ou 4 comprenant une étape g) dans laquelle au moins une partie de la fraction (H1), issue de l'étape e) ou de la fraction (H2) issue de l'étape f) est envoyée dans une zone de fractionnement (C4) permettant d'obtenir d'une part une fraction aqueuse (H3) appauvrie en alcool et d'autre part une fraction (A2) enrichie en alcool.

6. Procédé selon la revendication 5 dans lequel ladite fraction (A2) est au moins en partie recyclée dans la zone (R1) de l'étape a) de synthèse d'éther.

7. Procédé selon l'une des revendications 5 ou 6 dans lequel au moins une partie de la fraction aqueuse (H3) est envoyée dans la zone (L1) d'extraction à l'eau de l'étape l'étape e) ou dans la zone (L2) d'extraction à l'eau de l'étape f).

8. Procédé selon l'une des revendications 3 à 7 comprenant au moins un moyen d'introduction d'eau d'appoint dans la zone (L1) d'extraction par lavage à l'eau de l'étape e) dans le cas de la présence d'une telle zone (L1).

9. Procédé selon l'une des revendications 4 à 8 comprenant au moins un moyen d'introduction d'eau d'appoint dans la zone (L2) d'extraction par lavage à l'eau de l'étape f) dans le cas de la présence d'une telle zone (L2).

10. Procédé selon l'une des revendications 3 à 9 comprenant une étape h) dans le cas de la présence d'une zone (L1) réalisant l'étape e), dans laquelle au moins une partie de la fraction organique (O2) issue de la zone (L1) de l'étape e) est envoyée dans une zone de fractionnement (C2) à partir de laquelle on obtient une fraction (O3) contenant de l'alpha oléfine de haute pureté (αhp2) et une fraction (O5) appauvrie en alpha oléfine.

11. Procédé selon la revendication 10 comprenant une étape i) tel que au moins une partie de la fraction (O3) est envoyée dans une zone de fractionnement (C3) à partir de laquelle on obtient une fraction (O4) contenant l'alpha oléfine de haute pureté (ahp3) et une fraction (O6) contenant notamment des composés saturés initialement présents dans ladite partie de fraction (O3).

12. Procédé selon l'une des revendications 3 à 11 tel que, dans le cas de la présence d'une zone (L2) réalisant l'étape f), au moins une partie de la fraction (B2) est envoyée, dans une étape j), dans une zone de fractionnement (C6) à partir de laquelle on récupère une fraction (B3) contenant de l'oléfine tertiaire de haute pureté (Ohp2) et une fraction (B4) appauvrie en oléfine tertiaire.

13. Procédé selon l'une des revendications 1 à 12 comprenant une étape k) dans laquelle au moins une partie de la fraction (E1) issue de la zone (C1) de l'étape b) contenant de l'éther alkylique tertiaire est envoyée dans une zone de fractionnement (C7) à partir de laquelle on récupère une fraction (E2) enrichie en éther alkylique tertiaire et appauvrie en composé lourds et une fraction (Q) appauvrie en éther alkylique tertiaire et enrichie en composés lourds.

14. Procédé selon la revendication 13 dans lequel au moins une partie de la fraction (E2) enrichie en éther alkylique tertiaire est envoyée à l'étape c) dans la zone (R2) de décomposition dudit éther alkylique tertiaire.

15. Procédé selon l'une des revendications 13 ou 14 dans lequel au moins une partie de la fraction organique (E1) est envoyée aux fractions carburant moteur et une autre partie de ladite fraction organique (E1) est envoyée à l'étape k) de fractionnement.

16. Procédé selon l'une des revendications 1 à 15 dans lequel la zone de fractionnement (C1) comprend une zone de distillation réactive effectuant simultanément l'étape a) et l'étape b) dudit procédé.

17. Procédé selon l'une des revendications 3 à 16 dans lequel au moins une partie de la ou les fraction(s) organiques obtenue(s) à la sortie d'au moins une zone d'extraction par lavage à l'eau est , dans le cas de la présence d'au moins une zone (L1) réalisant l'étape e) de la revendication 3 associée à une zone (C2) réalisant l'étape h) de la revendication 10, ou (L2) réalisant l'étape f) de la revendication 4 associée à une zone (C6) réalisant l'étape j) de la revendication 12, est (sont) envoyée(s) dans une zone de séparation (Coe) à partir de laquelle on récupère une fraction liquide aqueuse (Lae) et une fraction liquide organique (Lbe) contenant la majeure partie de l'oléfine initialement présente dans ladite partie, ladite fraction (Lbe) appauvrie en eau et contenant ladite oléfine et éventuellement d'autres composés, étant alors envoyée respectivement dans les zones de fractionnement respectives (C2) et (C6) à partir desquelles on obtient respectivement de l'alpha oléfine purifiée (α'hp2) et de l'oléfine tertiaire purifiée (O'hp2).

## Patentansprüche

1. Verfahren zur Herstellung reinen alpha-Olefins, reinen tertiären Olefins, und gegebenenfalls tertiären Alkylethers ausgehend von einem Schnitt ungesättigter Kohlenwasserstoffe, der wenigstens ein alpha-Olefin und wenigstens ein tertiäres etherifizierbares Olefin enthält, wobei das Verfahren dadurch gekennzeichnet ist, dass es umfasst:
a) eine Stufe der Bildung wenigstens eines tertiären A1kylethers durch in Kontakt bringen von einem kohlenwasserstoffhaltigen Schnitt, der wenigstens ein alpha-Olefin und wenigstens ein tertiäres etherifizierbares Olefin enthält, mit wenigstens einem Alkohol in einer Reaktionszone (R1), die einen Etherifzierungskatalysator enthält,
b) eine Stufe der Fraktionierung wenigstens eines Teils des aus der Stufe a) stammenden Produkts in einer Zone der Fraktionierung (C1), die es ermöglicht, einerseits eine organische, an tertiärem Alkylether verarmte und das alpha-Olefin enthaltende Fraktion (O1) zu erhalten und in eine organische Fraktion (E1), die an dem während der Stufe a) gebildeten tertiären Alkylether angereichert ist, wobei die organische Fraktion (O1) später durch Waschen mit Wasser und dann Fraktionierung gereinigt wird,
c) eine Stufe der Zersetzung wenigstens eines Teils des tertiären Alkylethers, der in der organischen Fraktion (E1) der Stufe b) enthalten ist, in einer Reaktionszone (R2), die einen Zersetzungskatalysator dieses Ethers enthält, zu einem Produkt (P1), welches wenigstens einen Alkohol und wenigstens ein tertiäres Olefin enthält und
d) eine Stufe der Fraktionierung wenigstens eines Teils des aus der Stufe c) stammenden Produkts (P1) in einer Fraktionierungszone (C5), die es ermöglicht, einerseits eine Fraktion (B1) zu erhalten, die den größeren Teil des tertiären Olefins enthält und gegebenenfalls eine kleinere Fraktion Alkohol und gegebenenfalls leichte Verbindungen, die gegebenenfalls anfänglich in diesem Teil des Produkts (P1) enthalten sind und andererseits eine Fraktion (A1), die den größeren Teil des in der Stufe c) gebildeten Alkohols enthält, gegebenenfalls in der Stufe c) nicht zersetzten Ether und gegebenenfalls schwere Verbindungen, die anfänglich in diesem Teil des aus der Stufe c) stammenden Produkts (P1) enthalten sind, wobei diese Fraktion (B1) später durch Waschen mit Wasser und dann durch Fraktionierung gereinigt wird.

2. Verfahren nach Anspruch 1, in dem wenigstens ein Teil der Fraktion (A1) in die Zone (R1) geschickt wird.

3. Verfahren nach einem der Ansprüche 1 oder 2, welches eine Stufe e) umfasst, in der wenigstens ein Teil der organischen aus der Fraktionierungszone (C1) der Stufe b) stammenden Fraktion (O1) in eine Zone (L1) der Extraktion durch Waschen mit Wasser geschickt wird, ausgehend von der man eine wässrige Fraktion (H1) erhält, die Wasser und den größeren Teil des anfänglich in diesem Teil des Produkts (O1) vorliegenden Alkohols enthält, und eine Fraktion (O2), die den größeren Teil des anfänglich in diesem Teil des Produkts (O1) vorliegenden alpha-Olefins enthält, wobei die Fraktion (O2) das gereinigte alpha-Olefin (αhp1), Wasser und gegebenenfalls andere Kohlenwasserstoffverbindungen enthält und im wesentlichen frei von Alkohol ist.

4. Verfahren nach einem der Ansprüche 1 bis 3, das eine Stufe f) umfasst, in der wenigstens ein Teil der Fraktion (R1) in eine Waschzone (L2) mit Wasser geschickt wird, ausgehend von der man eine Fraktion (B2), die den größeren Teil des anfänglich in diesem Teil des Produkts (B1) vorliegenden tertiären Olefins enthält, wobei diese Fraktion (B2) das gereinigte tertiäre Olefin (Ohp1), Wasser und gegebenenfalls leichte Verbindungen enthält und im wesentlichen frei von Alkohol ist, und eine wässrige Fraktion (H2) gewinnt, die Wasser enthält und den größeren Teil des anfänglich in der der Fraktion des Produkts (B1) vorliegenden Alkohols enthält.

5. Verfahren nach einem der Ansprüche 3 oder 4, das eine Stufe g) umfasst, in der wenigstens ein Teil der aus der Stufe e) stammenden Fraktion (H1) oder der aus der Stufe
f) stammenden Fraktion (H2) in eine Fraktionierungszone (C4) geschickt wird, die es ermöglicht, einerseits eine wässrige, an Alkohol verarmte Fraktion (H3) zu erhalten und andererseits eine an Alkohol angereicherte Fraktion (A2).

6. Verfahren nach Anspruch 5, in dem die Fraktion (A2) wenigstens teilweise in die Zone (R1) der Ethersynthese der Stufe a) rezykliert wird.

7. Verfahren nach einem der Ansprüche 5 oder 6, in dem wenigstens ein Teil der wässrigen Fraktion (H3) in die Wasserextraktionszone (L1) der Stufe e) oder in die Wasserextraktionszone (L2) der Stufe f) geschickt wird.

8. Verfahren nach einem der Ansprüche 3 bis 7, das wenigstens ein Mittel zur Einführung zusätzlichen Wassers in die Extraktionszone (L1) durch Waschen mit Wasser der Stufe e) im Falle der Anwesenheit einer solchen Zone (L1) umfasst.

9. Verfahren nach einem der Ansprüche 4 bis 8, das wenigstens ein Mittel zur Einführung zusätzlichen Wassers in die Extraktionszone (L2) durch Waschen mit Wasser der Stufe f) im Falle der Anwesenheit einer solchen Zone (L2) umfasst.

10. Verfahren nach einem der Ansprüche 3 bis 9, das eine Stufe h) im Falle de Anwesenheit einer die Stufe e) verwirklichenden Zone (L1) umfasst, in der wenigstens ein Teil der organischen, aus der Zone (L1) der Stufe e) stammenden Fraktion (O2) in eine Fraktionierungszone (C2) geschickt wird, ausgehend von der man eine Fraktion (O3) erhält, die alpha-Olefin von hoher Reinheit (αhp2) und eine an alpha-Olefin verarmte Fraktion (O5) enthält.

11. Verfahren nach Anspruch 10, das eine Stufe i) umfasst, so dass wenigstens ein Teil der Fraktion (O3) in eine Fraktionierungszone (C3) geschickt wird, ausgehend von der man eine Fraktion (O4) erhält, die das alpha-Olefin hoher Reinheit (αhp3) und eine Fraktion (O6) enthält, die insbesondere gesättigte Verbindungen enthält, die anfänglich in diesem Teil der Fraktion (O3) vorliegen.

12. Verfahren nach einem der Ansprüche 3 bis 11, derart, dass im Fall des Vorhandenseins einer die Stufe f) verwirklichenden Zone (L2) wenigstens ein Teil der Fraktion (B2) in einer Stufe j) in eine Fraktionierungszone (C6) geschickt wird, ausgehend von der man eine Fraktion (B3) gewinnt, die tertiäres Olefin hoher Reinheit (Ohp2) enthält und eine an tertiärem Olefin verarmte Fraktion (B4).

13. Verfahren nach einem der Ansprüche 1 bis 12, das eine Stufe k) umfasst, in der wenigstens ein Teil der aus der Zone (C1) der Stufe b) stammenden Fraktion (E1), die tertiären Alkylether enthält, in eine Fraktionierungszone (C7) geschickt wird, ausgehend von der man eine Fraktion (E2), die an Alkylether angereichert und an schweren Verbindungen verarmt ist, und eine Fraktion (Q) gewinnt, die an tertiärem Alkylether verarmt und an schweren Verbindungen angereichert ist.

14. Verfahren nach Anspruch 13, in dem wenigstens ein Teil der an tertiärem Alkylether angereicherten Fraktion (E2) zur Stufe c) in die Zersetzungszone (R2) des tertiären Alkylethers geschickt wird.

15. Verfahren nach einem der Ansprüche 13 oder 14, in dem wenigstens ein Teil der organischen Fraktion (E1) zu den Motortreibstofffraktionen geschickt wird und ein anderer Teil dieser organischen Fraktion (E1) zur Fraktionierungsstufe k) geschickt wird.

16. Verfahren nach einem der Ansprüche 1 bis 15, in dem die Fraktionierungszone (C1) eine reaktive Destillationszone umfasst, die gleichzeitig die Stufe a) und die Stufe b) des Verfahrens ausführt.

17. Verfahren nach einem der Ansprüche 3 bis 16, in dem wenigstens ein Teil der organischen Fraktion oder Fraktionen, die am Ausgang wenigstens einer Extraktionszone durch Waschen mit Wasser erhalten wird (werden) im Falle der Anwesenheit wenigstens einer die Stufe e) des Anspruchs 3 verwirklichenden Zone (L1), die einer die Stufe h) des Anspruchs 10 verwirklichenden Zone (C2) oder einer die Stufe f) des Anspruchs 4 verwirklichenden Zone (L2), die einer die Stufe j) des Anspruchs 12 verwirklichenden Zone (C6) zugeordnet ist, in eine Trennzone (Coe) geschickt wird (werden), ausgehend von der man eine flüssige wässrige Fraktion (Lae) und eine flüssige organische Fraktion (Lbe) gewinnt, die den größeren Teil des anfänglich in diesem Teil vorliegenden Olefins enthält, wobei diese Fraktion (Lbe), die an Wasser verarmt ist und das Olefin und gegebenenfalls andere Verbindungen enthält, dann jeweils in die jeweiligen Fraktionierungszonen (C2) und (C6) geschickt wird, ausgehend von der man jeweils gereinigtes alpha-Olefin (α'hp2) und gereinigtes tertiäres Olefin (O'hp2) erhält.

## Claims

1. A process for the production of a pure alpha olefin, a pure tertiary olefin and optionally a tertiary alkyl ether from an unsaturated hydrocarbon cut containing at least one alpha olefin and at least one etherifiable tertiary olefin, said process being characterized in that it comprises:
a) a step for forming at least one tertiary alkyl ether by bringing an hydrocarbon cut containing at least one alpha olefin and at least one etherifiable tertiary olefin into contact with at least one alcohol in a reaction zone (R1) containing an etherification catalyst;
b) a step for fractionating at least a portion of the product from step a) in a fractionation zone (C1) to obtain an organic fraction (O1) which is depleted in tertiary alkyl ether and containing the alpha olefin, and an organic fraction (E1), enriched in the tertiary alkyl ether formed during step a) ; said organic fraction (O1) being later purified by water washing and then by fractionating.
c) a step for decomposing at least a portion of the tertiary alkyl ether contained in the organic fraction (E1) from step b) into a product (P1) containing at least one alcohol and at least one tertiary olefin, in a reaction zone (R2) containing a catalyst for decomposing said ether; and
d) a step for fractionating at least a portion of product (P1) from step c), in a fractionation zone (C5) to obtain a fraction (B1) containing the major portion of the tertiary olefin and possibly a minor fraction of alcohol and possibly light compounds which may be initially contained in said portion of product (P1), and a fraction (A1) containing the major portion of the alcohol formed in step c), possibly ether which has not decomposed in step c) and possibly heavy compounds initially contained in said portion of product (P1) from step c), said fraction (B1) being later purified by water washing and then by fractionating.

2. A process according to claim 1, in which at least a portion of fraction (Al) is sent to zone (R1).

3. A process according to claim 1 or claim 2, comprising a step e) in which at least a portion of the organic fraction (O1) from fractionation zone (C1) of step b) is sent to a water washing extraction zone (L1) from which an aqueous fraction (H1) containing water and the major portion of the alcohol initially present in said portion of product (O1) and a fraction (O2) containing the major portion of the alpha olefin initially present in said portion of product (O1) are obtained, said fraction (O2) containing said purified alpha olefin (αhp1), water, and possibly other hydrocarbon compounds and being substantially free of alcohol.

4. A process according to any one of claims 1 to 3, comprising a step f) in which at least a portion of fraction (B1) is sent to a water washing zone (L2) from which a fraction (B2) containing the major portion of the tertiary olefin initially present in said portion of product (B1) is recovered, said fraction (B2) containing said purified tertiary olefin (Ohpl), water, and possibly light compounds and being substantially free of alcohol, and an aqueous fraction (H2) containing water and the major portion of the alcohol initially present in said portion of product (B1) is recovered.

5. A process according to claim 3 or claim 4, comprising a step g) in which at least a portion of fraction (H1) from step e) or fraction (H2) from step f) is sent to a fractionation zone (C4) to obtain an aqueous fraction (H3) which is depleted in alcohol and a fraction (A2) which is enriched in alcohol.

6. A process according to claim 5, in which at least a portion of said fraction (A2) is recycled to the ether synthesis zone (R1) of step a).

7. A process according to claim 5 or claim 6, in which at least a portion of the aqueous fraction (H3) is sent to the water extraction zone (L1) of step e) or to water extraction zone (L2) of step f).

8. A process according to any one of claims 3 to 7, comprising at least one means for introducing makeup water into the water washing extraction zone (L1) of step e) when such a zone (L1) is present.

9. A process according to any one of claims 4 to 8, comprising at least one means for introducing makeup water into the water washing extraction zone (L2) of step f) when such a zone (L2) is present.

10. A process according to any one of claims 3 to 9, comprising a step h) when a zone (L1) carrying out step e) is present, in which at least a portion of the organic fraction (02) from zone (L1) of step e) is sent to a fractionation zone (C2) from which a fraction (O3) containing high purity alpha olefin (αhp2) and a fraction (O5) which is depleted in alpha olefin are obtained.

11. A process according to claim 10, comprising a step i) in which at least a portion of the fraction (O3) is sent to a fractionation zone (C3) from which a fraction (O4) containing high purity alpha olefin (αhp3) and a fraction (O6) containing saturated compounds which are initially present in said portion of fraction (O3) are obtained.

12. A process according to any one of claims 3 to 11, in which, when a zone (L2) carrying out step f) is present, at least a portion of fraction (B2) is sent, in a step j), to a fractionation zone (C6) from which a fraction (B3) containing high purity tertiary olefin (Ohp2) and a fraction (B4) which is depleted in tertiary olefin are recovered.

13. A process according to any one of claims 1 to 12, comprising a step k) in which at least a portion of fraction (E1) from zone (C1) of step b) containing the tertiary alkyl ether is sent to a fractionation zone (C7) from which a fraction (E2) which is enriched in tertiary alkyl ether and depleted in heavy compounds and a fraction (Q) which is depleted in tertiary alkyl ether and enriched in heavy compounds are recovered.

14. A process according to claim 13, in which at least a portion of fraction (E2) which is enriched in tertiary alkyl ether is sent to step c) to zone (R2) for decomposition of said tertiary alkyl ether.

15. A process according to claim 13 or claim 14, in which at least a portion of the organic fraction (E1) is sent to the motor fuel pool and a further portion of said organic fraction (E1) is sent to fractionation step k).

16. A process according to any one of claims 1 to 15, in which the fractionation zone (C1) comprises a reactive distillation zone carrying out step a) and step b) of said process simultaneously.

17. A process according to any one of claims 3 to 16, in which at least a portion of the organic fraction(s) obtained from the outlet from at least one water washing extraction zone is, when at least one zone (L1) carrying out step e) of claim 3 is present associated with a zone (C2) carrying out step h) of claim 10, or (L2) carrying out step f) of claim 4 associated with a zone (C6) carrying out step j) of claim 12, is (are) sent to a separation zone (Coe) from which an aqueous liquid fraction (Lae) is recovered and an organic liquid fraction (Lbe) containing the major portion of the olefin initially present in said portion is recovered; said fraction (Lbe) being depleted in water and containing said olefin and possibly other compounds, then being sent respectively to the respective fractionation zones (C2) and (C6) from which purified alpha olefin (α'hp2) and purified tertiary olefin (O'hp2) are respectively obtained.
